# EUROPEAN PATENT SPECIFICATION

(11) **EP 2 730 279 B1**
(45) Date of publication and mention of the grant of the patent: **22.07.2015**
(21) Application number: 12007638.5
(22) Date of filing: 09.11.2012
(51) Int. Cl.: A61K 9/16, A61K 9/20

(54) **Immediate release formulations of cinacalcet**
Cinacalcetformulierungen mit sofortiger Freisetzung
Formulations à libération immédiate de cinacalcet

(43) Date of publication of application: 14.05.2014
(73) Proprietor: K.H.S. Pharma Holding GmbH, 55218 Ingelheim am Rhein (DE)
(72) Inventor: Leitão Silva, Gabriel, 3020-229 Coimbra (PT); Cadonau, Stephanie, 55435 Gau-Algesheim (DE); Paulo Simões, Sérgio, 3000-145 Coimbra (PT); Drescher, Christian, 55218 Ingelheim am Rhein (DE)
(74) Representative: Bendele, Tanja

(56) References cited:
- WO-A1-2005/034928
- WO-A2-2008/027522
- WO-A2-2008/064202
- WO-A2-2012/071535

## Description

### Introduction

Cinacalcet is a calcium receptor-active compound which is approved for the treatment of secondary hyperparathyroidism resulting from chronical kidney insufficiency and for the treatment of hypercalcaemia in patients with parathyroid carcinoma.

Cinacalcet in the form of its hydrochloric acid addition salt (cinacalcet HCl) is marketed under the brand names Mimpara^{®} and Parareg^{®} in Europe and Sensipar^{®} in the US.

The chemical name of cinacalcet HCl is (R)-N-[1-(1-naphthyl)ethyl]-3-[3-(trifluoromethyl)phenyl]propan-1-amine Hydrochloride and has the following structure:

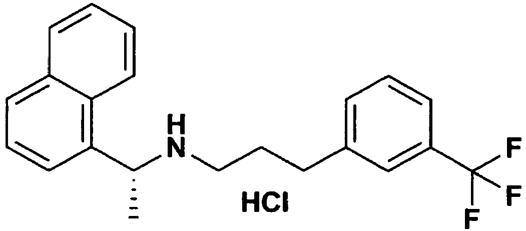

A synthesis of cinacalcet is disclosed in WO 96/12697. The manufacturing process of the most stable polymorphic form I of cinacalcet HCl is disclosed in WO 2007/62147.

The currently marketed tablets with immediate release of the drug are basically described in patent application WO 2005/034928.

In order to meet certain regulatory demands, WO 2005/034928 teaches the use of cinacalcet HCl compositions comprising:
(a) from 10% to 40% by weight of cinacalcet HCl
(b) from 45% to 85% by weight of at least one diluent;
(c) from 1% to 5% by weight of at least one binder;
(d) from 1% to 10% by weight of at least one disintegrant; and
(e) from 0.05% to 5% of at least one additive chosen from glidants, lubricants, and adherents; wherein the percentage by weight is relative to the total weight of the composition.

Patent application WO 2005/034928 reports a very low solubility of cinacalcet HCl in water of between 0.1mg/ml and 1.6mg/ml, depending on the pH value, resulting in a low bioavailability and limiting the formulation and delivery options available for this compound.

WO 2005/034928 also discloses a manufacturing process for cinacalcet formulations, indicating several "Critical Process Controls", i.e. parameters such as water level, impeller speed and water spray rate (during granulation), and blend time, tablet press speed, tablet weight, thickness, hardness and friability (during compression) etc. that might be adapted in order to achieve a desired result (i.e. meet the dissolution characteristics according to standards like USP 26/NF 21, chapter 711). As it appears from the application, also the quantitative composition of the tablets is a critical parameter which is meant to be adapted within the specified limits.

According to WO 2005/034928, cinacalcet HCl particles may have a D₅₀ of less or equal to about 50µm. However, the application remains silent about any technical implication related to the particle size of cinacalcet HCl particles. In addition, the application remains silent about the D₅₀ of the API that was actually used.

Patent application WO 2010/071689 suggests mechanical methods for particle size reduction of crystalline cinacalcet HCl in order to obtain particle sizes with a D₅₀ of less than or equal to 50µm, but also remains silent about any technical consequence in doing so, let alone providing any information of the behavior of cinacalcet HCl when formulated into pharmaceutical compositions.

WO 2010/034497 suggests that micronized cinacalcet HCl is hard to process and might be sensitive to oxidation. Example 4 of this application further indicates that formulations according to WO 2005/034928 with micronized cinacalcet HCl show inferior dissolution characteristics compared to those of non-micronized cinacalcet HCl with a D₅₀ of 101µm.

In conclusion, the prior art teaches to preferably use cinacalcet HCl with a D₅₀ of above 100µm, but remains silent about the implications of cinacalcet HCl particle sizes below 100µm for the manufacture of pharmaceutical formulations.

One object of the present invention therefore is the investigation of the correlation of the particle size of cinacalcet HCl and its dissolution characteristics.

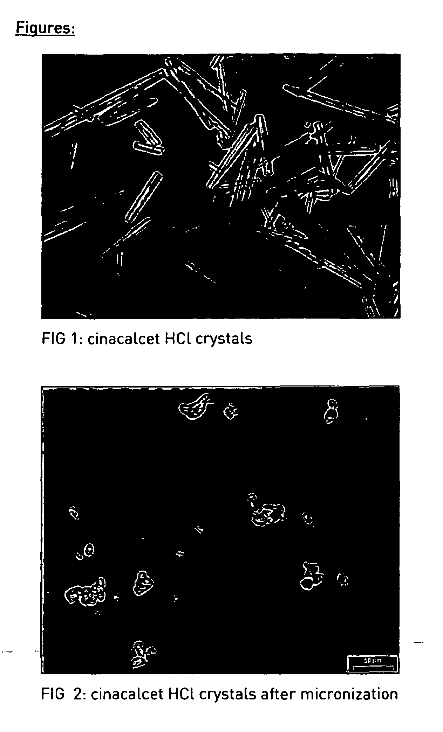

In accordance with the teaching of WO 2010/071689, crystallization of cinacalcet HCl under controlled conditions leads to large needle-shaped crystals, as can be seen in figure 1. Figure 2 illustrates a sample of micronized cinacalcet HCl.

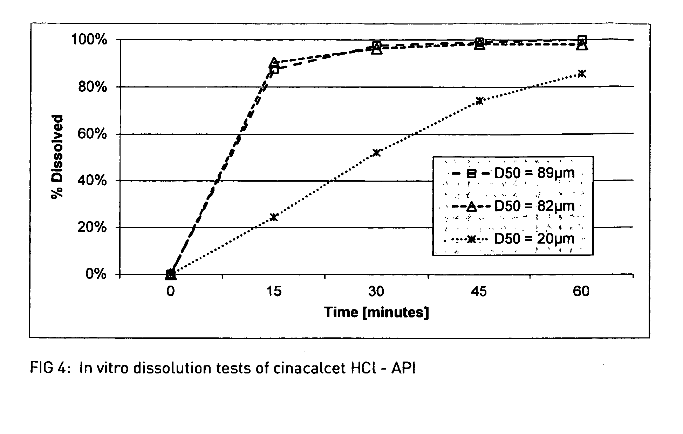

It has been found that dissolution of cinacalcet HCl API is heavily influenced by its D₅₀. A decrease of the dissolution rate with decreasing D₅₀ was observed (see figure 4).

Resulting from this observation, another object of the present invention is the provision of a formulation process which is flexible with respect to the use of cinacalcet HCl with particle sizes below 100µm.

In order obtain a market authorization for a generic drug development, it is essential to meet several regulatory requirements. Amongst them, the proof of bioequivalence is of major importance. The first step in aiming at bioequivalence is to approach the in-vitro dissolution profile of an existing market product. In order to do so, it is favorable to establish methods and processes that allow the selection and use of the active substance and all necessary ingredients and the adaption of relevant parameters within broad ranges. Therefore, the pharmaceutical formulation process shall be flexible over both a broad range of active drug load, but also with respect to the quantitative amounts of the excipients to be used.

All the abovementioned objectives have been solved by the formulation process and pharmaceutical compositions described hereinafter and the claims. It has been found that advantageous formulations comprise from 5.1% to 7% of one or more binders.

Binders such as povidone are usually not used in amounts of less than 0.5%, because of failure of binding activity. On the other hand, amounts above 5% are unusual since the strong binding activity might decrease the dissolution of the tablet. Now taking into consideration that cinacalcet HCl is extremely insoluble in water, it was a big surprise when the inventors of the present invention found out that cinacalcet HCl may be formulated with more than 5% binder without affecting the dissolution profile of the resulting formulation.

Pharmaceutical compositions according to the present invention further comprise
(a) from 15% to 50% cinacalcet HCl;
(b) from 33% to 80% of one ore more fillers;
(c) from 5.1% to 7% of one ore more binders;
and may comprise other pharmaceutical ingredients such as disintegrants, glidants or lubricants, wherein the percentage by weight is relative to the total weight of the composition.

The terms fillers, disintegrants, binders, lubricants, glidants etc. shall be understood as including a single compound, but also mixtures of compounds.

The preferred pharmaceutical composition is a tablet. Tablets may be manufactured according to processes well known in the art.

The cinacalcet HCl used in the pharmaceutical compositions is manufactured by known procedures indicated above, exhibiting a particle size with a D₅₀ ranging from 5µm to 95µm. Preferably, the D₅₀ ranges from 5µm to 45µm. More preferably, the D₅₀ ranges from 10µm to 30µm. Most preferred, the D₅₀-value ranges from 14µm to 23µm. The particle size of the cinacalcet HCl is measured according to light scattering techniques. The preferred crystal form is crystal form I as disclosed in WO 2007/62147.

Pharmaceutically acceptable fillers or diluents include starch, microcrystalline cellulose, dicalcium phosphate, lactose, calcium carbonate, magnesium carbonate, sorbitol, mannitol, sucrose, dextrine, kaolin, magnesium oxide, calcium sulfate, xylitol, isomalt, glucose, fructose, maltose, acids like citric acid, tartaric acid, fumaric acid, co-polymers such as those from vinyl pyrrolidone and vinyl acetate or those of polyethylene glycol, and mixtures thereof. Preferred diluents are pre-gelatinized maize starch and microcrystalline cellulose.

Pharmaceutically acceptable binders include povidone, hydroxypropyl methylcellulose, dihydroxy propylcellulose, sodium carboxyl methylcellulose, and mixtures thereof. Preferred binder is povidone.

Pharmaceutically acceptable disintegrants include sodium starch glycolate, crospovidone, croscarmellose sodium, and mixtures thereof. Preferred disintegrant is sodium starch glycolate.

Pharmaceutical compositions according to the present invention may also comprise glidants such as colloidal silicon dioxide.

Pharmaceutically acceptable lubricants include magnesium stearate, calcium stearate, stearic acid, stearic acid, glyceryl behenate, hexanedioic acid, hygrogenated vegetable oil sodium stearyl fumarate and glycerine fumarate. Preferred lubricant is magnesium stearate.

The tablets according to the invention may comprise further common pharmaceutically acceptable excipients and may be film coated.

In general, the formulation process comprises the following steps:
(a) The drug substance, binders, and portions of fillers and disintegrants are mixed and blended.
(b) The pre-mixture is then wet granulated;
(c) The wet granules are passed through a sieve;
(d) The sieved wet granules are dried and the dried granules are calibrated;
(e) Remaining portions of fillers, disintegrants and glidants are mixed and sieved to form the external phase.
(f) The external phase mixture is blended with the granules.
(g) The lubricant is sieved and blended with the mixture of granules and external phase to form the final blend which is used for production of tablets.

Specifically, the formulation process comprises the following steps:
(a) mixing and blending the cinacalcet HCl with povidone, pre-gelatinzed maize starch with portions of microcrystalline cellulose and sodium starch glycolate;
(b) the pre-mixture is then granulated with water.
(c) the wet granulate is passed through a sieve (1.4mm);
(d) granules are dried in a fluid bed drier until loss on drying is around 3%;
(e) the dried granules are calibrated through a 0.2mm sieve;
(f) the remaining portions of microcrystalline cellulose and sodium starch glycolate and colloidal silicon dioxide are mixed and sieved though 0.71 mm sieve to form the external phase;
(g) the external phase mixture is blended with the granules;
(h) Magnesium stearate is blended with the mixture of granules and external phase to form the final blend which is used for production of tablets.

Granules prepared in step (b) exhibited a D₅₀ ranging between 50µm and 150µm, measured by sieve analysis according to WO 2005/034928.

The dissolution profile of the formulations were measured according to standard dissolution protocols (USP paddle, 37°C+/- 0.5°C, 75 rpm, 0.05 N HCl, 900ml).

### Example 1: Dissolution characteristics of cinacalcet HCl

The particle size distribution of cinacalcet HCl was measured by laser diffraction analysis, using the equipment and procedure as displayed in table 1:

**Table 1**

| Apparatus | Malvern Mastersizer 2000 (MALVERN INSTRUMENTS) |
|---|---|
| Technique used | Dispersion method |
| Parameters | - Lens: Fourier lens (0.02-2000µm) |
| | - Laser beam wave length: blue light (ca. 475nm) and red light (ca. 650nm) |
| | - Sample unit: Hydro 2000MU (A) |
| | - Analysis model: polydisperse |
| | - Presentation: Standard-wet |
| Dispersion medium | n-Hexane |
| Surfactant | Soy Lecithine |
| Sample preparation / Procedure | i) Prepare a solution of soy lecithin in n-hexane (1% w/v). This solution will be used as suspension media. |
| | ii) Wet a sample of powder with some drops of suspension medium and mix with help of a spatula, until all the material is wet. |
| | iii) Add more suspension media (about 40-50ml). |
| | iv) Homogenize the suspension under magnetic stirring |
| | v) Under stirring, take some material and place it on the equipment until an obscuration between 8 and 40% is obtained. |

200mg of cinacalcet HCl of different D₅₀ values was dropped into a vessel containing 900ml of dissolution medium (0.1 N HCl) at 37.0 +/- 0.5°C and stirred at 75rpm. The results are displayed in table 2 and figure 4.

**Table 2**

| Time (min) | D₅₀=89µm | D₅₀=82µm | D₅₀=20µm |
|---|---|---|---|
| | % Dissolution* | | |
| 0 | 0 | 0 | 0 |
| 15 | 87 | 90 | 24 |
| 30 | 98 | 96 | 52 |
| 45 | 99 | 98 | 74 |
| 60 | 100 | 98 | 86 |

| | | | |
|---|---|---|---|
| * Dissolution rate of D₅₀=89 µm after 60min is set to 100% | | | |

The results clearly show that the cinacalcet HCl with a very low D₅₀ of 20µm exhibits a much lower solubility, indicating that the solubility of cinacalcet HCl decreases with decreasing particle size.

### Examples 2-5: Pharmaceutical formulations with cinacalcet HCl

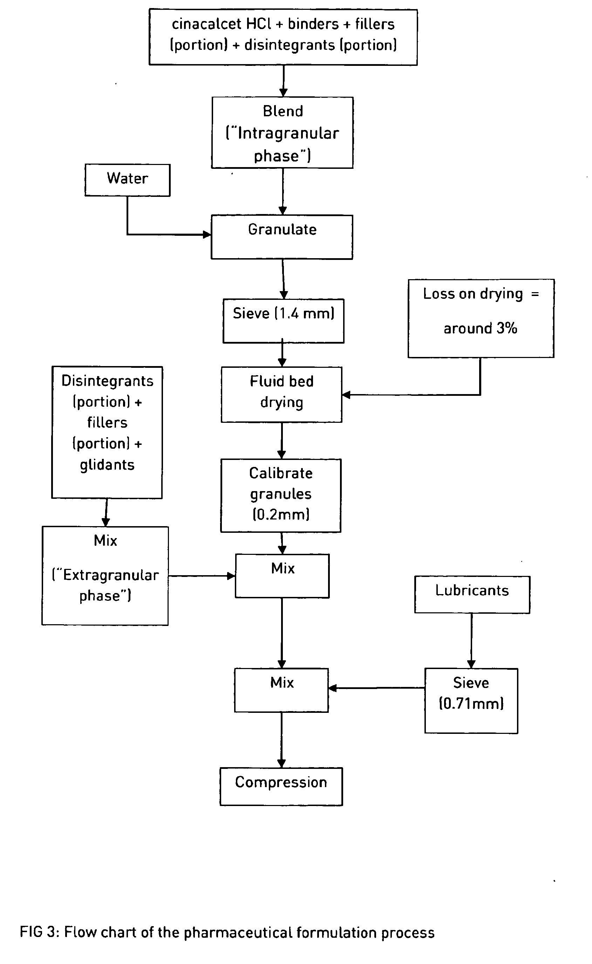

Tablets containing cinacalcet HCl were manufactured according to the process as shown in figure 3, using equipment and settings used in the manufacturing process as described in table 3.

**Table 3**

| **Stage** | **Machine/Description** | **Settings** |
|---|---|---|
| **Internal Phase - Mixture for Granulation** | | |
| Blending (Granulation pre-mixture) | ERWEKA - Double Cone Mixer (lab scale) | - |
| | Mixing time | 5min - 10min |
| Granulation | ERWEKA - Lab scale | - |
| | Povidone concentration | 6% solution w/w |
| | Granulation/blending time | 1min - 5min |
| | Grid size (to sieve the wet granules) | 1.4 mm |
| Drying | GLATT - Lab-scale mini-Glatt | - |
| | Inlet air temperature | 40°C - 55°C |
| | Product temperature | 40°C - 45°C |
| | Air pressure | 0.7bar |
| Granules calibration | HammerMill Knives | - |
| | Grid size (to sieve dry granules) | 0.2mm |
| Blending (External excipients) | ERWEKA - Double Cone Mixer (lab scale) | - |
| | Mixing time | 10min |

| **External Phase / Lubrication** | | |
|---|---|---|
| Blending (External Phase) | ERWEKA - Double Cone Mixer (lab scale) | - |
| | Mixing time | 15min |
| Blending (Final-Blend) | ERWEKA - Double Cone Mixer (lab scale) | - |
| | Grid size - to sieve the external phase/lubricants | 0.71 mm |
| | Mixing time | 3min |

The composition of the resulting formulation for a tablet comprising 30mg Cinacalcet is displayed table 4:

**Table 3**

| **Substances** | **Function** | **Examples 2 & 3** | | **Examples 4 & 5** | |
|---|---|---|---|---|---|
| | | **Percentage** | **Quantity (mg)** | **Percentage** | **Quantity (g)** |
| cinacalcet HCl | Drug Substance | 18.4% | 33.1 * | 44.0% | 33,1* |
| D₅₀ (drug substance particle) | | Example 2: 23 µm | | Example 4: 23 µm | |
| | | Example 3: 14 µm | | Example 5: 14 µm | |
| Pre-gelatinized Maize Starch (1500 - Colorcon) | Filler | 30.0% | 54.0 | 23.0% | 17.3 |
| Microcrystalline Cellulose (FMC 102) | Filler | 6.0% (Int¹⁾) | 10.8 | 5.0% (Int¹⁾) | 3.8 |
| | | 27.6% (Ext¹⁾) | 49.4 | 10.0% (Ext²⁾) | 7.5 |
| Povidone (Kollidon 25) | Binder | 6.0% | 10.8 | 6.0% | 4.5 |
| Sodium Starch Glycolate | Disintegrant | 5.0% (Int¹⁾) | 9.0 | 5.0% (Int¹⁾) | 3.8 |
| | | 6.0% (Ext²⁾) | 10.8 | 6.0% (Ext²⁾) | 4.5 |
| Colloidal Silicon dioxide (Aerosil 200) | Glidant | 0.5% | 0.9 | 0.5% | 0.38 |
| Magnesium Stearate | Lubricant | 0.5% | 0.9 | 0.5% | 0.38 |
| | TOTAL | 100.0% | 180 | 100.0% | |

| | | | | | |
|---|---|---|---|---|---|
| * corresponding to 30mg cinacalcet free base ¹⁾ intragranutar phase ²⁾ Extragranular phase | | | | | |

Tablets containing 60 mg and 90mg of cinacalcet HCl may be prepared accordingly by compressing the same mixture to tablets of double or triple tablet weight.

### Dissolution characteristics of the pharmaceutical compositions

The in-vitro dissolution of tablets (30mg) according to examples 2 to 5 were analyzed according to Ph. Eur. 2.9.3 and USP <711> - Method 2 (Paddle Apparatus) with the settings as displayed in table 5.

**Table 5**

| Equipment: | Varian |
|---|---|
| Medium: | HCl 0.05N |
| Volume: | 900ml |
| Temperature: | 37.0°C ± 0.5°C |
| Stirrinq speed: | 75 rpm |

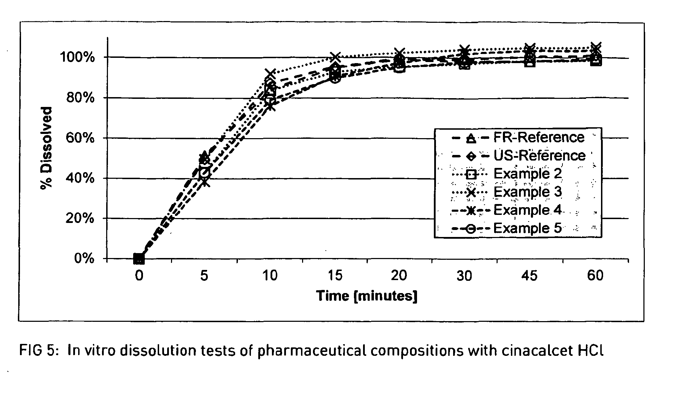

The dissolution profiles of the formulations are displayed in table 6 and figure 5. Unlike the dissolution experiments with unformulated cinacalcet HCl with a very small particle size, all tested compositions release at least about 85% of the cinacalcet HCl from the composition in no later than 30 minutes from the start of the test. Furthermore, comparison with the original products Mimpara^{®} from France (FR-Reference) and the United States (US-Reference) showed an almost identical dissolution profile.

**Table 6**

| | Example 2 | Example 3 | Example 4 | Example | FR-Reference | US-Reference |
|---|---|---|---|---|---|---|
| Time (min) | % Dissolution | | | | | |
| 0 | 0,0 | 0,0 | 0.0 | 0.0 | 0,0 | 0,0 |
| 5 | 43.1 | 49.9 | 38.5 | 43.5 | 51.4 | 49.7 |
| 10 | 83.7 | 91.9 | 76.1 | 79.1 | 84.2 | 87.4 |
| 15 | 92.9 | 100.2 | 90.8 | 90.1 | 95.1 | 95.4 |
| 20 | 95.5 | 102.3 | 97.3 | 95.1 | 99.1 | 99.4 |
| 30 | 97.2 | 104.1 | 102.1 | 98.7 | 98.3 | 99.2 |
| 45 | 98.2 | 104.7 | 103.4 | 99.8 | 98.0 | 100.3 |
| 60 | 98.7 | 105.1 | 103.6 | 99.9 | 99,7 | 100.9 |

### Stability testing of the pharmaceutical compositions

The tablets of example 3 were stored for 1 month period under standard accelerated temperature and relative humidity conditions (40°C/75%RH) and were then analyzed for assay and impurity profile. Conditions for assay and purity determination by HPLC were according to table 8.

| | |
|---|---|
| Analytical column: | Luna C5 (5µm, 250mm*4.6mm) |
| Mobile phase A: | Acetonitrile |
| Mobile phase B: | 0.02M Ammonium Acetate |
| Flow rate: | 1.0mL/min |
| Column oven temperature: | 30°C |
| Detection wavelength: | 220nm |

### Gradient:

**Table 7**

| Time | %A | %B |
|---|---|---|
| 0 | 55 | 45 |
| 20 | 70 | 30 |
| 25 | 70 | 30 |
| 33 | 95 | 5" |

No influence of temperature or moisture on the impurity profile of the tablets was observed. Additionally, there seems to be no influence of oxygen on the stability of the formulation, as derives from the results of experiments with open in comparison to closed flasks (see table 8).

**Table 8**

| | Specification | time=0 | time=1 month | |
|---|---|---|---|---|
| | | | closed flask | open flask |
| Total impurities (HPLC) | NMT 0.5% | ≤ 0.1% | ≤ 0.1% | ≤ 0.1% |
| Cinacalcet HCl Assay (HPLC) | 95.0 - 105.0% | 99.3% | 99.0% | 99.2% |

In conclusion, the present formulations exhibited no degradation of the drug substance, thus a good stability, indicating the robustness of the present formulation process and pharmaceutical compositions.

## Claims

1. Pharmaceutical composition, comprising:
(a) from 15 to 50% by weight cinacalcet HCl;
(b) from 30 to 80% by weight of one or more fillers;
(c) from 5.1% to 7% by weight of one or more binders;
and optionally one or more disintegrants, one or more glidants and/or one or more lubricants or one or more other acceptable pharmaceutical excipients, wherein the percentage by weight is relative to the total weight of the composition.

2. Pharmaceutical composition according to claim 1, comprising:
(b) from 35% to 70% by weight of one or more fillers.

3. Pharmaceutical composition according to claim 1 or 2, further comprising:
(d) from 10.1 % to 15% by weight of one or more disintegrants.

4. Pharmaceutical composition according to one of the preceding claims, comprising:
(a) from 18% to 45% by weight cinacalcet HCl;
(b) from 35% to 64% by weight of one or more fillers;
(c) from 5.1% to 7% by weight of one or more binders;
(d) from 10.1% to 12% by weight of one or more disintegrants.

5. Pharmaceutical composition according to one of the claims 1 to 4, comprising:
(a) from 18% to 20% by weight cinacalcet HCl;
(b) from 60% to 64% by weight of one or more fillers;
(c) from 5.1% to 7% by weight of one or more binders;
(d) from 10.1% to 12% by weight of one or more disintegrants.

6. Pharmaceutical composition according to one of the claims 1 to 4, comprising:
(a) from 41% to 44% by weight cinacalcet HCl;
(b) from 35% to 40% by weight of one or more fillers;
(c) from 5.1 % to 7% by weight of one or more binders
(d) from 10.1% to 12% by weight of one or more disintegrants

7. Pharmaceutical composition according to one of the claims 3 to 6, comprising:
(d) from 10.5% to 11.5% by weight of one or more disintegrants.

8. Pharmaceutical composition according to one of the claims 1 to 5, comprising an intragranular phase, comprising
(a) from 18% to 20% by weight cinacalcet HCl;
(b') from 30% to 40% by weight of one or more fillers;
(c) from 5.1% to 7% by weight of one or more binders;
(d) from 5% to 6% by weight of one or more disintegrants;
and an extragranular phase, comprising
(b") from 25% to 28% by weight of one or more fillers;
(d") from 5% to 6% by weight of one or more disintegrants;
(e) from 0.2% to 1% by weight of one or more glidants.

9. Pharmaceutical composition according to one of the claims 1 to 5, comprising an intragranular phase, comprising
(a) from 41% to 44% by weight cinacalcet HCl;
(b') from 25% to 30% by weight of one or more fillers;
(c) from 5.1% to 7% by weight of one or more binders;
(d') from 5% to 6% by weight of one or more disintegrants;
and an extragranular phase, comprising
(b") from 8% to 12% by weight of one or more fillers;
(d") from 5% to 6% by weight of one or more disintegrants;
(e) from 0.2% to 1% by weight of one or more glidants.

10. Pharmaceutical composition according to one of the preceding claims, comprising:
(c) from 5.5% to 6.5% by weight of one or more binders.

11. Pharmaceutical composition according to one of preceding claims, comprising:
(f) from 0.2% to 1.5% by weight of one or more lubricants.

12. Pharmaceutical composition according to one of the preceding claims, wherein the cinacalcet HCl particles exhibits a D₅₀ ranging from 5µm to 95µm, preferably ranging from 5µm to 45µm, more preferably ranging from 10µm to 30µm, even more preferably ranging from 14µm to 23µm.

13. Process for the manufacture of a pharmaceutical composition comprising cinacalcet HCl according to one of the preceding claims, comprising the following steps:
(a) Mixing cinacalcet HCl, the binders, a portion of the fillers and a portion of the disintegrants to form an intragranular phase;
(b) Wet granulating the intragranular phase to form wet granules;
(c) Sieving the wet granules;
(d) Drying the wet granules and calibrating the dried granules;
(e) Mixing and sieving the remaining portions of fillers and disintegrants with the glidants to form an extragranular phase;
(f) Blending the extragranular phase with the dried granules;
(g) Sieving the lubricants and blend them with the mixture of dried granules and the extragranular phase to form the final blend which is used for production of the tablets.

## Patentansprüche

1. Pharmazeutische Zusammensetzung, umfassend:
(a) 15 bis 50 Gewichtsprozent Cinacalcet HCl;
(b) 30 bis 80 Gewichtsprozent eines oder mehrerer Füllstoffe;
(c) 5,1 bis 7 Gewichtsprozent eines oder mehrerer Bindemittel;
und optional ein oder mehrere Sprengmittel, ein oder mehrere Fließregulierungsmittel und/oder ein oder mehrere Gleitmittel oder einen oder mehrere andere pharmazeutisch verträgliche Hilfsstoffe, wobei der Gewichtsprozentsatz bezogen auf das Gesamtgewicht der Zusammensetzung relativ ist.

2. Pharmazeutische Zusammensetzung nach Anspruch 1, umfassend:
(b) 35 bis 70 Gewichtsprozent eines oder mehrerer Füllstoffe.

3. Pharmazeutische Zusammensetzung nach Anspruch 1 oder 2, ferner umfassend:
(d) 10,1 bis 15 Gewichtsprozent eines oder mehrerer Sprengmittel.

4. Pharmazeutische Zusammensetzung nach einem der vorhergehenden Ansprüche, umfassend:
(a) 18 bis 45 Gewichtsprozent Cinacalcet HCl;
(b) 35 bis 64 Gewichtsprozent eines oder mehrerer Füllstoffe;
(c) 5,1 bis 7 Gewichtsprozent eines oder mehrerer Bindemittel;
(d) 10,1 bis 12 Gewichtsprozent eines oder mehrerer Sprengmittel.

5. Pharmazeutische Zusammensetzung nach einem der Ansprüche 1 bis 4, umfassend:
(a) 18 bis 20 Gewichtsprozent Clnacalcet HCl;
(b) 60 bis 64 Gewichtsprozent eines oder mehrerer Füllstoffe;
(c) 5,1 bis 7 Gewichtsprozent eines oder mehrerer Bindemittel;
(d) 10,1 bis 12 Gewichtsprozent eines oder mehrerer Sprengmittel.

6. Pharmazeutische Zusammensetzung nach einem der Ansprüche 1 bis 4, umfassend:
(a) 41 bis 44 Gewichtsprozent Cinacalcet HCl;
(b) 35 bis 40 Gewichtsprozent eines oder mehrerer Füllstoffe;
(c) 5,1 bis 7 Gewichtsprozent eines oder mehrerer Bindemittel;
(d) 10,1 bis 12 Gewichtsprozent eines oder mehrerer Sprengmittel.

7. Pharmazeutische Zusammensetzung nach einem der Ansprüche 3 bis 6, umfassend:
(d) 10,5 bis 11,5 Gewichtsprozent eines oder mehrerer Sprengmittel.

8. Pharmazeutische Zusammensetzung nach einem der Ansprüche 1 bis 5, umfassend eine intergranulare Phase, umfassend
(a) 18 bis 20 Gewichtsprozent Cinacalcet HCl;
(b') 30 bis 40 Gewichtsprozent eines oder mehrerer Füllstoffe;
(c) 5,1 bis 7 Gewichtsprozent eines oder mehrerer Bindemittel;
(d') 5 bis 6 Gewichtsprozent eines oder mehrerer Sprengmittel;
und eine extragranulare Phase, umfassend
(b") 25 bis 28 Gewichtsprozent eines oder mehrerer Füllstoffe:
(d") 5 bis 6 Gewichtsprozent eines oder mehrerer Sprengmittel;
(e) 0,2 bis 1 Gewichtsprozent eines oder mehrerer Fließregulierungsmittel.

9. Pharmazeutische Zusammensetzung nach einem der Ansprüche 1 bis 5, umfassend eine intergranulare Phase, umfassend
(a) 41 bis 44 Gewichtsprozent Cinacalcet HCl;
(b') 25 bis 30 Gewichtsprozent eines oder mehrerer Füllstoffe;
(c) 5,1 bis 7 Gewichtsprozent eines oder mehrerer Bindemittel;
(d') 5 bis 6 Gewichtsprozent eines oder mehrerer Sprengmittel;
und eine extragranulare Phase, umfassend
(b") 8 bis 12 Gewichtsprozent eines oder mehrerer Füllstoffe;
(d") 5 bis 6 Gewichtsprozent eines oder mehrerer Sprengmittel;
(e) 0,2 bis 1 Gewichtsprozent eines oder mehrerer Fließregulierungsmittel.

10. Pharmazeutische Zusammensetzung nach einem der vorhergehenden Ansprüche, umfassend:
(c) 5,5 bis 6,5 Gewichtsprozent eines oder mehrerer Bindemittel.

11. Pharmazeutische Zusammensetzung nach einem der vorhergehenden Ansprüche, umfassend:
(f) 0,2 bis 1,5 Gewichtsprozent eines oder mehrerer Gleitmittel.

12. Pharmazeutische Zusammensetzung nach einem der vorhergehenden Ansprüche, wobei die Cinacalcet HCl Teilchen eine mittlere Partikelgröße D50 im Bereich von 5 µm bis 95 µm aufweisen, bevorzugt im Bereich von 5 µm bis 45 µm, besonders bevorzugt im Bereich von 10 µm bis 30 µm, ganz besonders bevorzugt im Bereich von 14 µm bis 23 µm.

13. Verfahren zur Herstellung einer pharmazeutischen Zusammensetzung, die Cinacalcet HCl enthält, nach einem der vorhergehenden Ansprüche, umfassend die folgenden Schritte:
(a) Mischen von Cinacalcet HCl, den Bindemittels, einem Teil der Füllstoffe und einem Teil der Sprengmittel, um eine intergranulare Phase zu bilden;
(b) Feuchtgranulierung der intergranularen Phase, um Feuchtgranulat zu bilden;
(c) Sieben des Feuchtgranulats,
(d) Trocknen des Feuchtgranulats und Kalibrieren des getrockneten Granulats;
(e) Mischen und Sieben der verbleibenden Teile der Füllstoffe und Sprengmittel mit den Fließregulierungsmitteln, um eine extragranulare Phase zu bilden;
(f) Mischen der extragranularen Phase mit dem getrockneten Granulat;
(g) Sieben der Gleitmittel und Mischen der Gleitmittel mit der Mischung aus getrocknetem Granulat und extragranularer Phase, um die endgültige Mischung zu bilden, die zur Herstellung der Tabletten verwendet wird.

## Revendications

1. Composition pharmaceutique, comprenant :
(a) 15 à 50 % en poids de cinacalcet HCl ;
(b) 30 à 80 % en poids d'une ou plusieurs charges ;
(c) 5,1 à 7 % en poids d'un ou plusieurs liants ;
et éventuellement un ou plusieurs désintégrants, un ou plusieurs agents de glissement et / ou un ou plusieurs lubrifiants ou une ou plusieurs autres excipients pharmaceutiquement acceptables, dans lequel le pourcentage en poids est relatif par rapport au poids total de la composition.

2. Composition pharmaceutique selon la revendication 1, comprenant :
(b) 35 à 70 % en poids d'une ou plusieurs charges.

3. Composition pharmaceutique selon la revendication 1 ou 2, comprenant en outre :
(d) 10,1 à 15 % en poids d'un ou plusieurs désintégrants,

4. Composition pharmaceutique selon l'une quelconque des revendications précédentes, comprenant :
(a) 18 à 45 % en poids de cinacalcet HCl ;
(b) 35 à 64 % en poids d'une ou plusieurs charges ;
(c) 5,1 à 7 % en poids d'un ou plusieurs liants ;
(d) 10,1 à 12 % en poids d'un ou plusieurs désintégrants.

5. Composition pharmaceutique selon l'une quelconque des revendications 1 à 4, comprenant :
(a) 18 à 20 % en poids de cinacalcet HCl ;
(b) 60 à 64 % en poids d'une ou plusieurs charges ;
(c) 5,1 à 7 % en poids d'un ou plusieurs liants ;
(d) 10,1 à 12 % en poids d'un ou plusieurs désintégrants.

6. Composition pharmaceutique selon l'une quelconque des revendications 1 à 4. comprenant :
(a) 41 à 44 % en poids de cinacalcet HCl ;
(b) 35 à 40 % en poids d'une ou plusieurs charges ;
(c) 5,1 à 7 % en poids d'un ou plusieurs liants ;
(d) 10,1 à 12 % en poids d'un ou plusieurs désintégrants.

7. Composition pharmaceutique selon l'une quelconque des revendications 3 à 6, comprenant :
(d) 10,5 à 11,5 % en poids d'un ou plusieurs désintégrants.

8. Composition pharmaceutique selon l'une quelconque des revendications 1 à 5, comprenant une phase intergranulaire, comprenant
(a) 18 à 20 % en poids de cinacalcet HCl ;
(b') 30 à 40 % en poids d'une ou plusieurs charges ;
(c) 5,1 à 7 % en poids d'un ou plusieurs liants ;
(d') 5 à 6 % en poids d'un ou plusieurs désintégrants ;
et une phase extragranulaire, comprenant
(b") 25 à 28 % en poids d'une ou plusieurs charges ;
(d") 5 à 6 % en poids d'un ou plusieurs désintégrants ;
(e) 0,2 à 1 % en poids d'un ou plusieurs agents de glissement.

9. Composition pharmaceutique selon l'une quelconque des revendications 1 à 5, comprenant une phase intergranulaire, comprenant
(a) 41 à 44 % en poids de cinacalcet HCl ;
(b') 25 à 30 % en poids d'une ou plusieurs charges ;
(c) 5,1 à 7 % en poids d'un ou plusieurs liants ;
(d') 5 à 6 % en poids d'un ou plusieurs désintégrants ;
et une phase extragranulaire, comprenant
(b") 8 à 12 % en poids d'une ou plusieurs charges ;
(d") 5 à 6 % en poids d'un ou plusieurs désintégrants ;
(e) 0,2 à 1 % en poids d'un ou plusieurs agents de glissement.

10. Composition pharmaceutique selon l'une quelconque des revendications précédentes, comprenant :
(c) 5,5 à 6,5 % en poids d'un ou plusieurs liants.

11. Composition pharmaceutique selon l'une quelconque des revendications précédentes, comprenant :
(f) 0,2 à 1,5 % en poids d'un ou plusieurs lubrifiants.

12. Composition pharmaceutique selon l'une quelconque des revendications précédentes, dans lequel les particules de cinacalcet HCl présentent une taille moyenne de particule D50 allant de 5 µm à 95 µm, de préférence allant de 5 µm à 45 µm, de manière davantage préférée allant de 10 µm à 30 µm, encore plus préférablement allant de 14 µm à 23 µm.

13. Procédé pour la fabrication d'une composition pharmaceutique comprenant du cinacalcet selon l'une quelconque des revendications précédentes, comprenant les étapes suivantes :
(a) le mélange du cinacalcet HCl, les liants, une partie des charges et une partie des désintégrants pour former une phase intergranulaire ;
(b) la granulation humide de la phase intergranulaire pour former des granulés humides ;
(c) le tamisage des granulés humides ;
(d) le séchage des granulés humides et le calibrage des granulés séchés ;
(e) le mélange et le tamisage des parties restantes des charges et des désintégrants avec les agents de glissement pour former une phase extragranulaire ;
(f) le mélange de la phase extragranulaire avec les granulés sèches ;
(g) le tamisage des lubrifiants et le mélange des lubrifiants avec le mélange de granulés séchés et la phase extragranulaire pour former le mélange final qui est utilisé pour la production des comprimés.
